# EUROPEAN PATENT APPLICATION

(11) **EP 2 003 112 A1**
(43) Date of publication of application: **17.12.2008**
(21) Application number: 07740249.3
(22) Date of filing: 29.03.2007
(51) Int. Cl.: C07C 51/373, C07C 49/233, C07C 59/56, C07C 69/738, C07C 45/65, C07C 51/353, C07C 51/367, C07C 59/88, C07C 67/313, C07C 67/343

(54) **METHOD FOR PRODUCING CARBOXYLIC ACID COMPOUND**

(30) Priority: 31.03.2006 JP 2006096334
(71) Applicant: Kowa Company, Ltd., Naka-ku Nagoya-shi, Aichi-ken 460-8625 (JP)
(72) Inventor: KATSURADA, Manabu, Yokohama-shi, Kanagawa; 227-8502 (JP); NAKAJIMA, Yasuko, Yokohama-shi, Kanagawa; 227-8502 (JP); KASUGA, Yuzo, Kitakyusyu-shi, Fukuoka; 806-0004 (JP); ONOGI, Kazuhiro, Iruma-shi, Saitama 358-0023 (JP); ODA, Toshiaki, Higashimurayama-shi Tokyo 189-0014 (JP)
(74) Representative: Polypatent
(86) International application number: PCT/JP2007/056811
(87) International publication number: WO 2007/114223

(57) **Abstract**

A method for conveniently preparing 2,2-dichloro-12-(4-chlorophenyl)-10-hydroxydodecanoic acid useful as an active ingredient of therapeutic agents for diseases such as diabetes at a high yield, which comprises the step of reacting a compound represented by the following general formula (A): 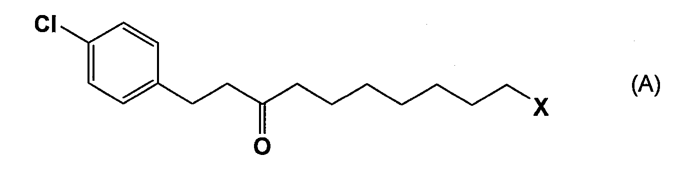 wherein X represents a halogen atom, and a compound represented by the following general formula (B): CHCl₂COOR¹ wherein R¹ represents hydrogen atom or a protective group of carboxyl group, to prepare a compound represented by the following general formula (C): 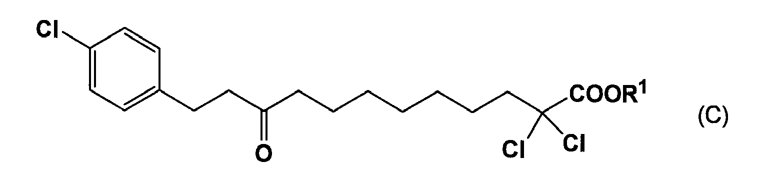 wherein R¹ has the same meaning as that defined above.

## Description

### Technical Field

The present invention relates to a method for preparing 2,2-dichloro-12-(4-chlorophenyl)-10-hydroxydodecanoic acid and synthetic intermediates thereof.

### Background Art

Carboxylic acid compounds represented by the following formula: 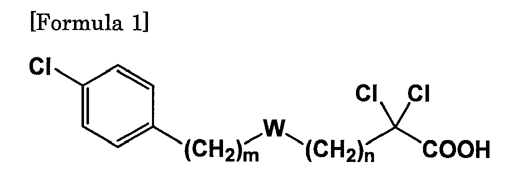 wherein m represents an integer of 0 to 4, n represents an integer of 5 to 9, and W represents -CH(OR^{a})- (R^{a} represents hydrogen atom or a protective group of hydroxyl group) or -C(=O)-, are known to have potent hypoglycemic action, plasma insulin decreasing action, and triglyceride decreasing action, and can exhibit superior prophylactic and/or therapeutic action against diseases including diabetes, diabetic complications, hyperlipidemia, arteriosclerosis, and the like without weight gain or obesity (Patent document 1).

In Patent document 1, one of the compounds represented by the aforementioned formula, 2,2-dichloro-12-(4-chlorophenyl)-10-hydroxydodecanoic acid is prepared by reacting 4-chlorophenylethyl magnesium bromide (I), prepared from 4-(2-bromoethyl)chlorobenzene and magnesium, with 8-bromo-1-octanal to prepare 10-bromo-1-(4-chlorophenyl)-3-decanol (II), then reacting the resulting compound with acetyl chloride to prepare 3-acetoxy-10-bromo-1-(4-chlorophenyl)decane (III), then reacting the resulting compound with methyl dichloroacetate to prepare methyl 10-acetoxy-2,2-dichloro-12-(4-chlorophenyl)dodecanoate (IV), and further performing deprotection.

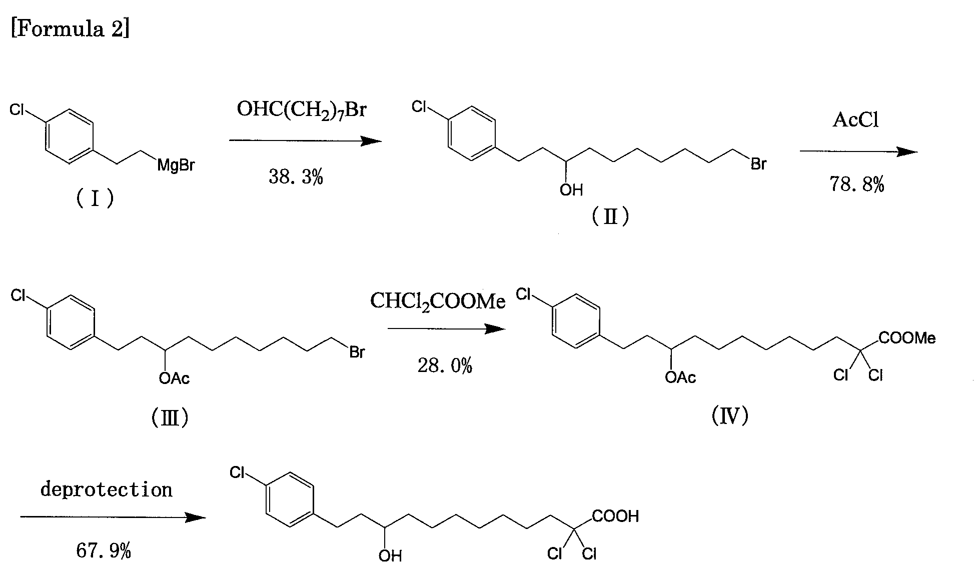

However, all the synthetic intermediates produced in the steps of the aforementioned preparation method (II, III and IV) are oily substances, and accordingly, the method has a problem in that complicated operations such as column chromatography are required for isolation and purification of the products. Moreover, due to low yields of the aforementioned steps for preparing the synthetic intermediates (II) and (IV), a total yield for all the steps is as high as 5.7%, and accordingly, the method is disadvantageous as an industrial preparation method of 2,2-dichloro-12-(4-chlorophenyl)-10-hydroxydodecanoic acid. Therefore, it has been desired to develop a method for preparing the target compound with more convenient operation in a high yield.
Patent document 1: International Patent Publication WO2004/103946

### Disclosure of the Invention

### Object to be Achieved by the Invention

An object of the present invention is to provide a method for conveniently preparing 2,2-dichloro-12-(4-chlorophenyl)-10-hydroxydodecanoic acid in a high yield.
Another object of the present invention is to provide synthetic intermediates useful for conveniently preparing 2,2-dichloro-12-(4-chlorophenyl)-10-hydroxydodecanoic acid in a high yield.

### Means for Achieving the Object

In order to achieve the aforementioned objects, the inventors of the present invention conducted various researches on methods for preparing 2,2-dichloro-12-(4-chlorophenyl)-10-hydroxydodecanoic acid, and as a result, inventors found that the target compound was successfully obtained conveniently and in a high yield by a preparation method in which said compound is prepared via novel synthetic intermediates (A) and (D) as shown in the reaction formulas described below.

The present invention thus provides a method for preparing 2,2-dichloro-12-(4-chlorophenyl)-10-hydroxydodecanoic acid, which comprises the step of reacting a compound represented by the following general formula (A): 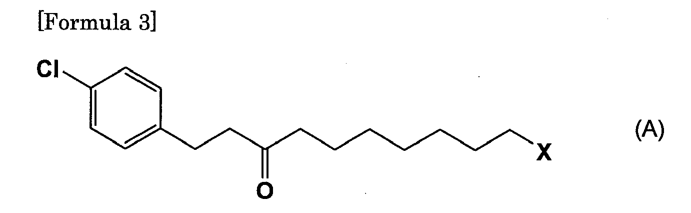 wherein X represents a halogen atom, and a compound represented by the following general formula (B):

CHCl₂COOR¹

wherein R¹ represents hydrogen atom or a protective group of carboxyl group, to prepare a compound represented by the following general formula (C): 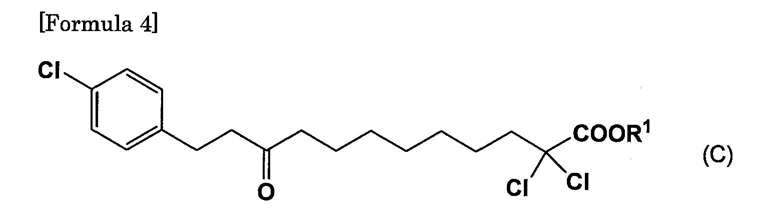 wherein R¹ has the same meaning as that defined above. In the aforementioned reaction, as the protective group of carboxyl group represented by R¹, preferably an alkyl group, more preferably a lower alkyl group having about 1 to 6 carbon atoms, most preferably methyl group, can be used, and as X, chlorine atom or iodine atom is preferred.

According to a preferred embodiment of the present invention, there is provided the aforementioned method, comprising the step of reacting a compound of the general formula (A) wherein X is iodine atom and a compound of the general formula (B) wherein R¹ is an alkyl group to prepare a compound of the general formula (C) wherein R¹ is an alkyl group. There are also provided the aforementioned method, which further comprises the step of reacting a compound of the general formula (A) wherein X is chlorine atom with an iodinating agent to prepare a compound of the general formula (A) wherein X is iodine atom; the aforementioned method, which further comprises the step of performing deprotection of the compound of the general formula (C) wherein R¹ is a protective group of carboxyl group, preferably an alkyl group, to prepare a compound of the general formula (C) wherein R¹ is hydrogen atom; and the aforementioned method, which further comprises the step of reducing the compound of the general formula (C) wherein R¹ is hydrogen atom to prepare 2,2-dichloro-12-(4-chlorophenyl)-10-hydroxydodecanoic acid.

According to more preferred embodiments of the present invention, there are provided the aforementioned method, wherein the compound represented by the general formula (A) is a compound prepared by subjecting a compound represented by the following general formula (D): 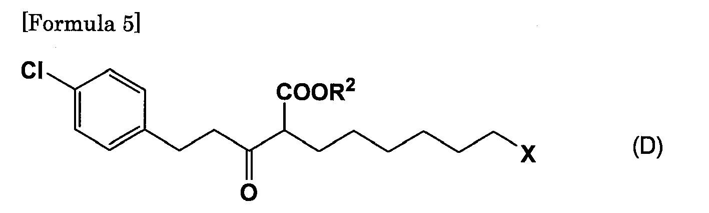 wherein X has the same meaning as that defined above, and R² represents a protective group of carboxyl group, to a decarboxylation reaction; the aforementioned method, wherein the compound represented by the general formula (D) is a compound obtained by reacting a compound represented by the following general formula (E): 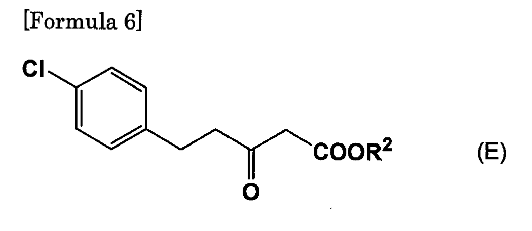 wherein R² has the same meaning as that defined above, and a 1,6-dihalogenohexane; and the aforementioned method, wherein 1-bromo-6-chlorohexane is used in the aforementioned reaction.

According to a more preferred embodiment of the aforementioned invention, there is provided the method for preparing 2,2-dichloro-12-(4-chlorophenyl)-10-hydroxydodecanoic acid, which comprises the following steps:
(1) the step of reacting a compound of the general formula (A) wherein X is chlorine atom and an iodinating agent to prepare a compound of the general formula (A) wherein X is iodine atom;
(2) the step of reacting the compound obtained in the above step (1) and a compound of the aforementioned general formula (B) wherein R¹ is a protective group of carboxyl group to prepare a compound of the aforementioned general formula (C) wherein R¹ is a protective group of carboxyl group; and
(3) the step of performing deprotection of the compound obtained in the above step (2).

Further, according to a still more preferred embodiment, there is provided the method for preparing 2,2-dichloro-12-(4-chlorophenyl)-10-hydroxydodecanoic acid, which comprises the following steps:
(1) the step of subjecting a compound of the general formula (D) wherein X is chlorine atom to a decarboxylation reaction to prepare a compound of the general formula (A) wherein X is chlorine atom;
(2) the step of reacting the compound obtained in the above step (1) and an iodinating agent to prepare a compound of the aforementioned general formula (A) wherein X is iodine atom;
(3) the step of reacting the compound obtained in the above step (2) and a compound of the aforementioned general formula (B) wherein R¹ is a protective group of carboxyl group to prepare a compound of the aforementioned general formula (C) wherein R¹ is a protective group of carboxyl group; and
(4) the step of performing deprotection of the compound obtained in the above step (3).

As a particularly preferred embodiment, the present invention also provides the method for preparing 2,2-dichloro-12-(4-chlorophenyl)-10-hydroxydodecanoic acid, which comprises the following steps:
(1) the step of reacting a compound represented by the aforementioned general formula (E) with 1-bromo-6-chlorohexane in the presence of a base to prepare a compound of the aforementioned general formula (D) wherein X is chlorine atom;
(2) the step of subjecting the compound obtained in the above step (1) to a decarboxylation reaction to prepare a compound of the general formula (A) wherein X is chlorine atom;
(3) the step of reacting the compound obtained in the above step (2) and an iodinating agent to prepare a compound of the aforementioned general formula (A) wherein X is iodine atom;
(4) the step of reacting the compound obtained in the above step (3) and a compound of the aforementioned general formula (B) wherein R¹ is a protective group of carboxyl group to prepare a compound of the aforementioned general formula (C) wherein R¹ is a protective group of carboxyl group; and
(5) the step of performing deprotection of the compound obtained in the above step (4) to prepare a compound of the aforementioned general formula (C) wherein R¹ is hydrogen atom.

According to the most preferred embodiment, the present invention also provides a method for preparing 2,2-dichloro-12-(4-chlorophenyl)-10-hydroxydodecanoic acid, which comprises the following steps:
(1) the step of reacting a compound represented by the aforementioned general formula (E) with 1-bromo-6-chlorohexane in the presence of a base to prepare a compound of the aforementioned general formula (D) wherein X is chlorine atom;
(2) the step of subjecting the compound obtained in the above step (1) to a decarboxylation reaction to prepare a compound of the general formula (A) wherein X is chlorine atom;
(3) the step of reacting the compound obtained in the above step (2) and an iodinating agent to prepare a compound of the aforementioned general formula (A) wherein X is iodine atom;
(4) the step of reacting the compound obtained in the above step (3) and a compound of the aforementioned general formula (B) wherein R¹ is a protective group of carboxyl group to prepare a compound of the aforementioned general formula (C) wherein R¹ is a protective group of carboxyl group;
(5) the step of performing deprotection of the compound obtained in the above step (4) to prepare a compound of the aforementioned general formula (C) wherein R¹ is hydrogen atom; and
(6) the step of reducing the compound obtained in the above step (5) to prepare 2,2-dichloro-12-(4-chlorophenyl)-10-hydroxydodecanoic acid.

From other aspects, the present invention provides a compound represented by the aforementioned general formula (A), preferably a compound represented by the aforementioned general formula (A) wherein X is chlorine atom or iodine atom; and a compound represented by the aforementioned general formula (D), preferably a compound represented by the aforementioned general formula (D) wherein X is chlorine atom, and R² is an alkyl group, particularly preferably a compound represented by the aforementioned general formula (D) wherein X is chlorine atom, and R² is ethyl group.

### Effect of the Invention

According to the method of the present invention, 2,2-dichloro-12-(4-chlorophenyl)-10-hydroxydodecanoic acid useful as a medicament for prophylactic and/or therapeutic treatment of diseases including diabetes, diabetic complications, hyperlipidemia, arteriosclerosis, and the like can be conveniently prepared in a high yield. Also according to the present invention, a compound represented by the aforementioned general formula (A) as a synthetic intermediate can be isolated and purified as crystalline solid, and therefore operation efficiency can be remarkably improved in the method of the present invention as compared with the purification attained by column chromatography performed in the conventional method. Furthermore, an yield of each step is also high, and therefore a total yield of the whole method is also markedly improved as compared with the conventional method. Best Mode for Carrying out the Invention

In the compound represented by the general formula (A), chlorine atom or iodine atom is preferred as X. In the compound represented by the general formula (D), chlorine atom is preferred as X.
In the compounds represented by the general formula (B) and (C), the protective group of carboxyl group represented by R¹ is not particularly limited, and any protective group removable under an appropriate condition may be used. Protective groups of carboxyl group are described in, for example, T. W. Green & P.G.M. Wuts, Protective Groups in Organic Synthesis (John Wiley & Sons, Inc.), and the like, and those skilled in the art can easily choose an appropriate protective group. For example, an alkyl group, preferably a lower alkyl group having about 1 to 6 carbon atoms, can be used as the protective group. Although the alkyl group may be any of linear, branched and cyclic alkyl groups and an alkyl group consisting of a combination of those, a linear or branched alkyl group is preferred. More specifically, methyl group, ethyl group, n-propyl group, isopropyl group, n-butyl group, isobutyl group, sec-butyl group, tert-butyl group, pentyl group, hexyl group, and the like are preferred, and ethyl group and methyl group are more preferred. Particularly preferred is methyl group. In the compounds represented by the general formula (D) and (E), the protective group of carboxyl group represented by R² is the same as the above-explained protective group.

The present invention will be explained below with reference to the most preferred embodiment as explained above. However, the method of the present invention is not limited to this specific embodiment. The following explanations are those for exemplification, and the scope of the present invention is not limited to the details of the following explanations. In the following scheme, the steps of the aforementioned preferred embodiment are indicated.

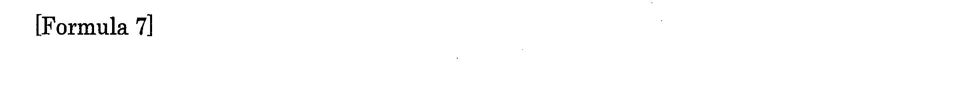

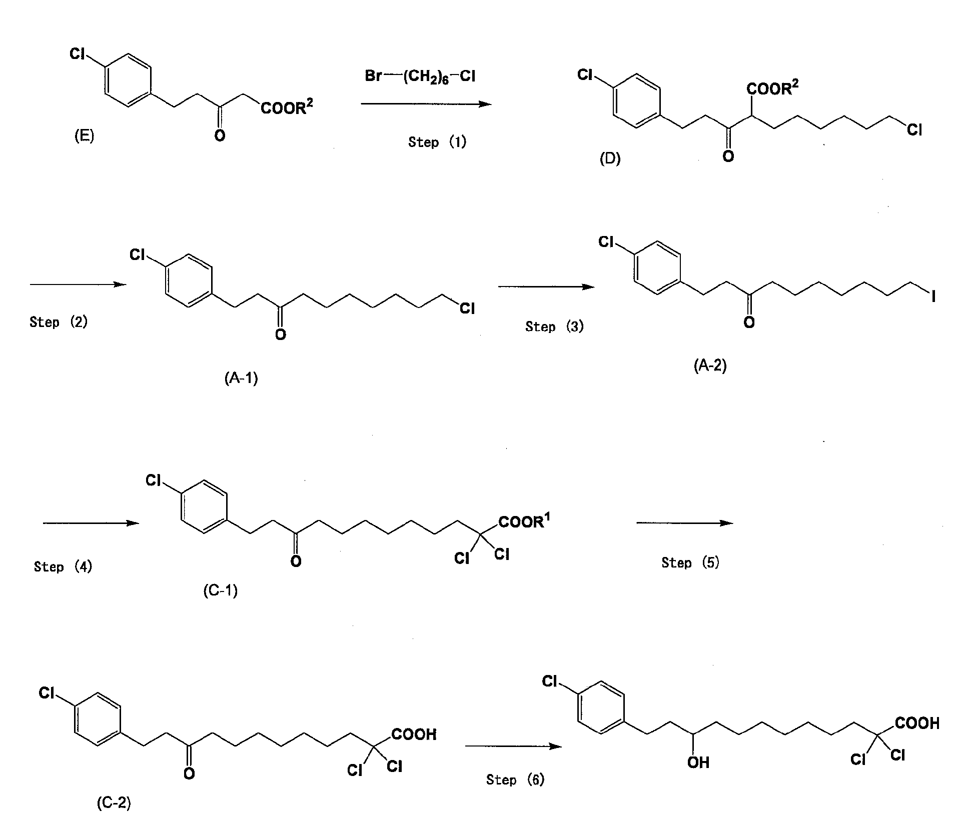

The step (1) is a method of reacting the compound (E) of which carboxyl group is protected and 1-bromo-6-chlorohexane to prepare a compound (D). This step can be typically performed by using an alkyl group as the protective group of carboxyl group, i.e., reacting a ketoester compound (E) as the compound (E) with 1-bromo-6-chlorohexane. This preferred embodiment is explained below. However, the method of the present invention is not limited to the use of an alkyl group as the protective group.
The reaction of the step (1) can be performed, for example, in a solvent in the presence of a base. The ketoester compound (E) can be prepared by, for example, the methods described in J. Heterocyclic Chem., 17 (2), 359-368 (1980); Synthesis, (18), 2844-2850, (2003), or similar methods.

In the aforementioned reaction, an amount of 1-bromo-6-chlorohexane is 1.0 to 3.0 moles, preferably 1.0 to 1.2 moles, based on 1 mole of the ketoester compound (E). Examples of the base include, for example, alkali metal hydrides such as sodium hydride and potassium hydride, lithium amide compounds such as lithium isopropylamide and lithium hexamethyldisilazide, alkali metal alkoxides such as sodium methoxide, potassium methoxide and potassium tert-butoxide and the like, and alkali metal alkoxides such as potassium tert-butoxide are preferred. Two or more kinds of these bases may be used in combination. Amount of the base used is 0.5 to 2.0 moles, preferably 1.0 to 1.2 moles, based on 1 mole of the ketoester compound (E).

The aforementioned step (1) is preferably performed in the presence of a solvent. The solvent to be used is not particularly limited so long as the solvent does not inhibit the reaction. Examples include, for example, amides such as N,N-dimethylformamide, N,N-dimethylacetamide and N-methylpyrrolidone, ureas such as N,N-dimethylimidazolidinone, sulfoxides such as dimethyl sulfoxide, sulfones such as sulfolane, ethers such as diethyl ether, diisopropyl ether, tetrahydrofuran and dioxane, aromatic hydrocarbons such as benzene, toluene and xylene, alcohols such as methanol, ethanol and tert-butanol, and ketones such as acetone and methyl isobutyl ketone, and alcohols such as tert-butanol and ketones such as methyl isobutyl ketone are particularly preferred. Two or more kinds of these solvents may be used in combination. Although an amount of the solvent used may be suitably chosen with considering homogeneousness of the reaction or efficiency of stirring, the amount is, for example, about 0.1 to 100 kg, preferably about 2.0 to 10 kg, based on 1.0 kg of the ketoester compound (E).

The aforementioned step (1) can be performed by a method of mixing the ketoester compound (E), 1-bromo-6-chlorohexane, the base and the solvent and performing the reaction with stirring, or the like. Although reaction temperature is not particularly limited, the temperature is, for example, -100 to 150°C, preferably 0°C to a temperature around a boiling point of a solvent, more preferably a temperature around a boiling point of a solvent.
In order to promote the reaction of the step (1), a catalyst such as potassium iodide can be used. Although an amount of potassium iodide used is not particularly limited, the amount is, for example, about 0.01 to 10 moles, preferably about 0.1 to 0.5 mole, based on 1.0 mole of the ketoester compound (E).
After completion of the reaction, the reaction mixture per se containing the product may be used for the reaction of the step (2), without performing complicated isolation and purification. If necessary, the reaction of the step (2) may also be performed after the target substance of the step (1) is isolated and purified by usual methods such as filtration, neutralization, extraction, concentration, distillation, and column chromatography.

The aforementioned step (2) is a reaction for decarboxylating the compound (D) obtained in the aforementioned step (1) to prepare a compound (A-1) (X is chlorine atom). This reaction can be performed by removing the protective group of carboxyl group of the compound (D) and subjecting the resultant to decarboxylation. For example, when a ketoester compound (D) is used according to the aforementioned preferred embodiment, the step (2) can be performed by removing the protective group of carboxyl group by hydrolysis in a solvent in the presence of a catalyst, and then decarboxylating the α-ketocarboxylic acid optionally with adding an acid or the like, if needed. In this case, if an acid is used as a catalyst for the hydrolysis, decarboxylation advances simultaneously, or if a base is used as a catalyst for the hydrolysis, after completion of the hydrolysis reaction, decarboxylation can be attained by adding an acid and warming the reaction mixture. The deprotection reaction can be suitably chosen according to type of the protective group of carboxyl group by those skilled in the art with referring to, for example, the book of Green et al. mentioned above. Use of an alkyl group as the protective group of carboxyl group is explained below. However, the method of the present invention is not limited to this preferred embodiment.

As the catalyst used for the hydrolysis, for example, an acid or a base is desirable. Examples of the acid include, for example, mineral acids such as sulfuric acid, hydrochloric acid, nitric acid and phosphoric acid, organic carboxylic acids such as formic acid, acetic acid and trifluoroacetic acid, and organic sulfonic acids such as methanesulfonic acid, trifluoromethanesulfonic acid, ethanesulfonic acid, benzenesulfonic acid and p-toluenesulfonic acid, and examples of the base include, for example, alkali metal hydroxides such as sodium hydroxide and potassium hydroxide, alkali metal alkoxides such as sodium methoxide and potassium methoxide, alkali metal hydrides such as sodium hydride and potassium hydride, alkali metal carbonates such as sodium carbonate and potassium carbonate, and the like. Alkali metal hydroxides can be preferably used. Although an amount of the catalyst used is not particularly limited, the amount is, for example, about 0.01 to 20 moles, preferably about 1 to 10 moles, based on 1 mole of the ketoester compound (D).
Examples of the acid used for the decarboxylation include the aforementioned acids. An amount thereof used is the same as that mentioned above.

The solvent to be used is not particularly limited, so long as the solvent does not inhibit the reaction, and for example, a mixture of water and any one of the solvents mentioned for the step (1) can be used. Preferred examples include alcohols such as methanol, ethanol and tert-butanol, and tert-butanol is particularly preferred. Two or more kinds of those solvents may be used in combination. Although an amount of the solvent used may be suitably chosen with considering homogeneousness of the reaction or efficiency of stirring, the amount is, for example, about 0.1 to 100 kg, preferably about 2.0 to 10 kg, based on 1.0 kg of the ketoester compound (D).
The aforementioned step (2) can be performed by a method of mixing the ketoester compound (D), the catalyst, and the solvent, and then performing the reaction with stirring, or the like. Although reaction temperature is not particularly limited, the temperature is, for example, 0 to 150°C, preferably 20°C to a temperature around a boiling point of a solvent, more preferably a temperature around a boiling point of a solvent.
After completion of the reaction, the chloro compound (A-1) per se obtained by the aforementioned step (2) may be used for the reaction of the step (3), without performing complicated isolation and purification. If necessary, the reaction of the step (3) may also be performed after the chloro compound (A-1) is isolated and purified by ordinary methods such as filtration, neutralization, extraction, concentration, distillation, and column chromatography.

The step (3) is a reaction of the chloro compound (A-1) (compound of the general formula (A) wherein X is chlorine atom) obtained in the aforementioned step (2) with an iodinating agent to perform a halogen exchange and thereby prepare a corresponding iodo compound (A-2) (compound of the general formula (A) wherein X is iodine atom).
Examples of the iodinating agent include, for example, sodium iodide, potassium iodide, and the like. An amount of the iodinating agent used is about 0.01 to 100 moles, preferably about 0.1 to 10 moles, more preferably about 1.0 to 3.0 moles, based on 1.0 mole of the chloro compound (A-1) as the starting material.

This reaction is preferably performed in the presence of a solvent. The solvent to be used is not particularly limited so long as the solvent does not inhibit the reaction, and the solvents explained for the step (1) can be used independently or in any combination of two or more kinds thereof. It is particularly preferable to use ketones such as methyl isobutyl ketone independently. Although an amount of the solvent used may be suitably chosen with considering homogeneousness of the reaction or efficiency of stirring, the amount is, for example, about 0.1 to 100 kg, preferably about 2.0 to 10 kg, based on 1.0 kg of the chloro compound (A-1) as the starting material.

The aforementioned step (3) is performed by a method of mixing the chloro compound (A-1) as the starting material and the iodinating agent in a solvent and performing the reaction with stirring, or the like. Although reaction temperature is not particularly limited, the temperature is, for example, -100 to 150°C, preferably 0°C to a temperature around a boiling point of a solvent, more preferably a temperature around a boiling point of a solvent.
The iodo compound (A-2) obtained by the aforementioned step (3) can be easily isolated by crystallization after completion of the reaction, and it is not necessary to perform any complicated operation such as chromatography and distillation for isolation and purification. Therefore, the method of the present invention utilizing this iodo compound as a synthetic intermediate is extremely advantageous in respect of simplification of the reaction operation and the like as compared with the conventional method. However, the iodo compound (A-2) may be separated by using ordinary isolation and purification means such as filtration, neutralization, extraction, concentration, distillation, recrystallization, and column chromatography, and then used for the reaction of the following step (4), as required.

The step (4) is a reaction of the iodo compound (A-2) with dichloroacetic acid represented by the general formula (B) of which carboxyl group is protected to obtain a compound (C-1) of which carboxyl group is protected. This step can be typically performed by using a dichloroacetic acid ester as the dichloroacetic acid represented by the general formula (B) of which carboxyl group is protected to obtain an ester compound as the compound (C-1). This preferred embodiment is explained below. However, the method of the present invention is not limited to this embodiment using an ester group as the protective group of carboxyl group.

Although the dichloroacetic acid ester (B) to be used can be suitably chosen according to the protective group of carboxyl group, a dichloroacetic acid alkyl ester can be preferably used, for example. More specifically, examples include, for example, methyl dichloroacetate, ethyl dichloroacetate, isopropyl dichloroacetate, n-butyl dichloroacetate, and the like, and methyl dichloroacetate is preferred. Although an amount of the dichloroacetic acid ester (B) used is not particularly limited, the amount is, for example, 0.5 to 10 moles, preferably 2.0 to 8.0 moles, based on 1 mole of the iodo compound (A-2).

It is preferable to use a base in the aforementioned step (4). As the base, for example, bases explained for the step (1) can be used, and it is preferable to use sodium hydride. Two or more kinds of bases may be used in combination. Although an amount of the base used is not particularly limited, the amount is, for example, about 0.5 to 2.0 moles, preferably about 1.0 to 1.2 moles, based on 1 mole of the iodo compound (A-2).
The aforementioned step (4) is desirably performed in the presence of a solvent. The solvent to be used is not particularly limited so long as the solvent does not inhibit the reaction, and for example, the solvents explained for the step (1) can be used. Amides such as N,N-dimethylformamide are particularly preferred. Two or more kinds of solvents may be used in combination. Although an amount of the solvent used may be suitably chosen with considering homogeneousness of the reaction or efficiency of stirring, the amount is, for example, about 0.1 to 100 kg, preferably about 2.0 to 10 kg, based on 1.0 kg of the iodo compound (A-2).

The aforementioned step (4) is performed by a method of mixing the iodo compound (A-2), the dichloroacetic acid ester (B), the base, and the solvent, and then performing the reaction with stirring, or the like. Although reaction temperature is not particularly limited, the temperature is, for example, about -100 to 150°C, preferably about -50 to 25°C, more preferably about -20 to 25°C.
The ester compound (C-1) obtained by the aforementioned step (4) can be used as the starting material of the following step (5) without performing any complicated isolation and purification. However, the ester compound (C-1) may be isolated and purified by using ordinary methods such as filtration, neutralization, extraction, concentration, distillation, and column chromatography, as required, and then used in the step (5).

The step (5) is a deprotection of the compound (C-1) of which carboxyl group is protected to obtain a carboxylic acid compound (C-2). The deprotection reaction can be suitably chosen according to the type of the protective group of carboxyl group by those skilled in the art with referring to, for example, the book of Green et al. mentioned above. The reaction where an alkyl group is used as the protective group of carboxyl group is explained below. However, the method of the present invention is not limited to the preferred embodiment.

According to a preferred embodiment of the aforementioned deprotection reaction, deprotection can be attained by performing hydrolysis in the presence of a catalyst such as an acid or a base. As the catalyst to be used, an acid or a base is preferred, and examples include, for example, the acids and bases explained for the step (2). Alkali metal hydroxides such as sodium hydroxide can be preferably used. An amount of the catalyst used is 0.01 to 20 moles, preferably 1 to 10 moles, based on 1 mole of the ester compound (C-1).
The solvent to be used is not particularly limited, so long as the solvent does not inhibit the reaction, and for example, a mixture of water and any one of the solvents mentioned for the step (1) can be used. Preferred examples include alcohols such as methanol, ethanol and tert-butanol, and methanol is particularly preferred.

The aforementioned step (5) can be performed by a method of mixing the ester compound (C-1), the catalyst, and the solvent, and performing the reaction with stirring, or the like. Although reaction temperature is not particularly limited, the temperature is, for example, 0 to 150°C, preferably 20°C to a temperature around the boiling point of the solvent, more preferably about 20 to 40°C.
The carboxylic acid compound (C-2) obtained by the aforementioned step (5) can be easily isolated as crystalline solid by crystallization after completion of the reaction, and accordingly, the target compound with high purity can be easily isolated without performing complicated isolation and purification operation such as chromatography, and can be used for the reaction of the following step (6). However, the target compound may be isolated and purified by using ordinary methods such as filtration, neutralization, extraction, concentration, distillation, recrystallization, and column chromatography, as required, and then used as a starting material of the reaction of the step (6).

The step (6) is a reduction of the carboxylic acid compound (C-2) to obtain the target compound, 2,2-dichloro-12-(4-chlorophenyl)-10-hydroxydodecanoic acid. The reaction can be typically performed by allowing a reducing agent to react on the carboxylic acid compound in a solvent.
As the reducing agent, for example, metal hydride compounds such as sodium borohydride and sodium cyanoborohydride can be used, and sodium borohydride is preferred. Although an amount of the reducing agent used is not particularly limited, the amount is, for example, about 0.5 to 10 moles, preferably about 1.0 to 4.0 moles, based on 1 mole of the carboxylic acid compound (C-2).

The solvent to be used is not particularly limited so long as the solvent does not inhibit the reaction, and for example, the solvents explained for the step (1) can be used. Two or more kinds of those solvents may be used in combination. For example, ethers such as tetrahydrofuran and dioxane, and alcohols such as methanol, ethanol and tert-butanol are preferred. Although an amount of the solvent used is not particularly limited and may be suitably chosen with considering homogeneousness of the reaction or efficiency of stirring, the amount is, for example, about 0.1 to 100 kg, preferably about 2.0 to 50 kg, based on 1.0 kg of the compound (C-2).

The aforementioned step (6) is performed by a method of mixing the carboxylic acid compound (C-2), the reducing agent, and the solvent, and then performing the reaction with stirring, or the like. Although reaction temperature is not particularly limited, the temperature is, for example, about -100 to 150°C, preferably about -50 to 25°C, more preferably about -20 to 10°C.
2,2-Dichloro-12-(4-chlorophenyl)-10-hydroxydodecanoic acid obtained by the aforementioned step (6) can be isolated by crystallization after completion of the reaction. The target compound may also be isolated and purified by ordinary methods such as filtration, neutralization, extraction, concentration, distillation, recrystallization, and column chromatography, as required.

In the method of the present invention, the aforementioned step (3) may be omitted, and the reaction of the step (4) may be performed by using the chloro compound (A-1) as a starting material and an iodinating agent as a catalyst. As the iodinating agent, the iodinating agents explained for the aforementioned step (3) can be used, and an amount as a catalyst is, for example, about 0.0001 to 0.01 mole based on 1.0 mole of the chloro compound (A-1) as the starting material.
By appropriately choosing reaction conditions of the method of the present invention, the compound (A) can also be prepared from the compound (E) by the steps (1) and (2) in one pot reaction without isolating the compound (D), or the carboxylic acid compound (C-2) can be prepared from the iodo compound (A-2) by the steps (4) and (5) in one pot reaction without isolating the compound (C-1). Such simplification of the process steps can be suitably applied by those skilled in the art, and it should be understood that the method in which the steps are simplified also fall within the scope of the present invention.

### Examples

The present invention will be more specifically explained with reference to examples. However, the scope of the present invention is not limited by the following examples.

### Example 1: Preparation of 2,2-dichloro-12-(4-chlorophenyl)-10-hydroxydodecanoic acid (1) Preparation of 10-chloro-1-(4-chlorophenyl)decan-3-one

Under a nitrogen atmosphere, a mixed solution of ethyl 5-(4-chlorophenyl)-3-oxopentanoate (3.02 kg, purity: 85.3 wt %), sodium iodide (0.3 kg), potassium tert-butoxide (1.25 kg), tert-butanol (10.25 kg) and methyl isobutyl ketone (5.19 kg) was warmed, and added dropwise with a mixed solution of 1-bromo-6-chlorohexane (2.22 kg) and methyl isobutyl ketone (5.19 kg) over 1 hour under reflux, and the mixture was stirred for 9 hours under reflux. The mixture was cooled to 33 to 37°C, and added dropwise with a mixture of sodium hydroxide (0.81 kg) and water (2.58 kg), and the mixture was stirred at 33 to 37°C for 5 hours. Then, the mixture was added dropwise with 35% hydrochloric acid (4.22 kg), and the mixture was stirred at 33 to 37°C for 4 hours. The mixture was added with water (12.81 kg), and the layers were separated. The organic layer was washed successively with a mixture of sodium hydrogencarbonate (0.9 kg) and water (12 kg), a mixture of sodium thiosulfate (0.646 kg) and water (12.81 kg), and a mixture of sodium chloride (3.23 kg) and water (9.71 kg), the solvent was evaporated, then the residue was subjected to thin film distillation, and the first fraction was removed (100°C, 2 mmHg) to obtain 10-chloro-1-(4-chlorophenyl)decan-3-one (2.99 kg, purity: 65.1 wt %) as brown oil (yield: 64%).
A part of the brown oil obtained was collected, and purified by column chromatography, and appearance and analytical data of 10-chloro-1-(4-chlorophenyl)decan-3-one were determined.
Appearance: White crystal
Melting point: 23.9-25.1°C
NMR (400MHz, CDCl₃) δ 1.20-1.34 (m, 4H), 1.36-1.46 (m, 2H), 1.50-1.60 (m, 2H), 1.70-1.80 (m, 2H), 2.37 (t, 2H, J = 7.6Hz), 2.70 (t, 2H, J = 7.6Hz), 2.86 (t, 2H, J = 7.6Hz), 3.52 (t, 2H, J = 7.6Hz), 7.09-7.13 (m, 2H), 7.21-7.26 (m, 2H)

It was able to isolate the intermediate obtained in the aforementioned reaction step, 10-chloro-1-(4-chlorophenyl)-4-ethoxycarbonyldecan-3-one, and the crude product was purified by column chromatography to obtain the compound as colorless oil. NMR (400MHz, CDCl₃) δ 1.22 (t, 3H, J = 7.1Hz), 1.24-1.44 (m, 6H), 1.70-1.88 (m, 4H), 2.68-2.93 (m, 4H), 3.37 (t, 1H, J = 7.3Hz), 3.51 (t, 2H, J = 6.6Hz), 4.13 (q, 2H, J = 7.1Hz), 7.11 (d, 2H, J = 8.3Hz), 7.23 (d, 2H, J = 8.3Hz)

### (2) Preparation of 1-(4-chlorophenyl)-10-iododecan-3-one

Under nitrogen atmosphere, a mixed solution of 10-chloro-1-(4-chlorophenyl)decan-3-one (2.96 kg, purity: 65.1 wt %), sodium iodide (3.04 kg) and methyl isobutyl ketone (19.55 kg) was warmed, and stirred for 12 hours under reflux. The mixture was cooled to 20 to 25°C, and added with n-heptane (4.15 kg) and water (12.81 kg), and the layers were separated. The organic layer was washed successively with a mixture of sodium thiosulfate (0.646 kg) and water (12.27 kg), and then with water (12.81 kg), and the solvent was evaporated. The residue was added with isopropyl alcohol (6.08 kg), then the mixture was cooled to 5°C, and the deposited crystals were collected by filtration. The crystals were washed with cold isopropyl alcohol (3.14 kg), and dried under reduced pressure to obtain 1-(4-chlorophenyl)-10-iododecan-3-one (2.19 kg, purity: 81.9 wt %) as pale brown crystals (yield: 72%). A part of the resulting pale brown crystals were collected and purified by column chromatography, and appearance and analytical data of 1-(4-chlorophenyl)-10-iododecan-3-one were determined.
Appearance: White crystal
Melting point: 44.5-46.5°C
NMR (400MHz, CDCl₃) δ 1.20-1.42 (m, 6H), 1.50-1.59 (m, 2H), 1.75-1.85 (m, 2H), 2.37 (t, 2H, J = 7.6Hz), 2.70 (t, 2H, J = 7.6Hz), 2.86 (t, 2H, J = 7.6Hz), 3.18, (t, 2H, J = 7.6Hz), 7.09-7.14 (m, 2H), 7.21-7.26 (m, 2H)

### (3) Preparation of methyl 2,2-dichloro-12-(4-chlorophenyl)-10-oxododecanoate

Under nitrogen atmosphere, sodium hydride (0.732 kg) and N,N-dimethylformamide (15.8 kg) were cooled to -5 to 0°C, and added dropwise with a mixture of 1-(4-chlorophenyl)-10-iododecan-3-one (2.19 kg, purity: 81.9 wt %), methyl dichloroacetate (2.62 kg) and N,N-dimethylformamide (15.8 kg) at an internal temperature below 15°C, and the mixture was stirred at -5 to 5°C for 4 hours. The mixture was added dropwise with a mixture of 35% hydrochloric acid (2.96 kg) and water (11.43 kg), and then added with n-heptane (10.2 kg) and toluene (6.46 kg), the layers were separated, and the organic layer was washed with water. The organic layer washed successively with a mixture of sodium hydrogencarbonate (0.652 kg) and water (8.65 kg), a mixture of sodium thiosulfate (0.465 kg) and water (8.84 kg), and then with water (9 kg), and the solvent was evaporated to obtain methyl 2,2-dichloro-12-(4-chlorophenyl)-10-oxododecanoate (2.41 kg, purity: 65.7 wt %) as pale brown oil (yield: 85%).

### (4) Preparation of 2,2-dichloro-12-(4-chlorophenyl)-10-oxododecanoic acid

Under nitrogen atmosphere, a mixture of methyl 2,2-dichloro-12-(4-chlorophenyl)-10-oxododecanoate (2.41 kg, purity: 65.7 wt %) and methanol (7.89 kg) was added dropwise with a mixture of sodium hydroxide (0.73 kg) and water (1.91 kg), and the mixture was stirred at 20 to 30°C for 4 hours. Then, the reaction mixture was added with toluene (6.24 kg), and the layers were separated. The resulting aqueous layer was made acidic by adding a mixture of 35% hydrochloric acid and water (10.84 kg), and then added with toluene (18.73 kg), and the layers were separated. The organic layer was washed twice successively with a mixture of methanol (5.92 kg) and water (7.40 kg), and a mixture of sodium chloride (1.44 kg) and water (14.4 kg). The organic layer was added with activated carbon (0.18 kg), the mixture was stirred, then the activated carbon was removed by filtration, and the solvent was evaporated. The residue was added with ethyl acetate (1.8 kg) and n-hexane (6.8 kg), and dissolved, the solution was cooled to 5°C, and the deposited crystals were collected by filtration, washed with a mixture of ethyl acetate (0.45 kg) and n-hexane (1.70 kg), and dried under reduced pressure to obtain 2,2-dichloro-12-(4-chlorophenyl)-10-oxododecanoic acid (1.34 kg, purity: 76.4 wt %) as pale brown crystals (yield: 67%).

### (5) Preparation of 2,2-dichloro-12-(4-chlorophenyl)-10-hydroxydodecanoic acid

Under a nitrogen atmosphere, a mixture of sodium borohydride (0.29 kg) and tetrahydrofuran (13.3 kg) was cooled to 5 to 10°C, and added dropwise with a mixture of 2,2-dichloro-12-(4-chlorophenyl)-10-oxododecanoic acid (1.31 kg, purity: 76.4 wt%) and ethanol (4.16 kg), and the mixture was stirred at 20 to 25°C for 1 hour. The reaction mixture was cooled to 5 to 10°C, added dropwise with a mixture of 35% hydrochloric acid (1.58 kg) and water (14.8 kg), and the mixture was extracted with toluene (10.4 kg). The organic layer was washed twice with water (12 kg), and the solvent was evaporated. Then, the residue was added with toluene (1.80 kg), the mixture was warmed to 20 to 25°C for dissolution, and the deposited crystals were collected by filtration, and dried under reduced pressure to obtain 2,2-dichloro-12-(4-chlorophenyl)-10-hydroxydodecanoic acid (1) (0.80 kg, purity: 97.3 wt %) as white crystals (yield: 77%).
By the steps (1) to (5) described above, it was able to obtain the target compound, 2,2-dichloro-12-(4-chlorophenyl)-10-hydroxydodecanoic acid from the starting material, ethyl 5-(4-chlorophenyl)-3-oxopentanoate, in a total yield of 20.2%. Industrial Applicability

According to the method of the present invention, 2,2-dichloro-12-(4-chlorophenyl)-10-hydroxydodecanoic acid useful as a medicament for prophylactic and/or therapeutic treatment of diseases including diabetes, diabetic complications, hyperlipidemia, arteriosclerosis and the like can be conveniently prepared in a high yield.

## Claims

1. A method for preparing 2,2-dichloro-12-(4-chlorophenyl)-10-hydroxydodecanoic acid, which comprises the step of reacting a compound represented by the following general formula (A): 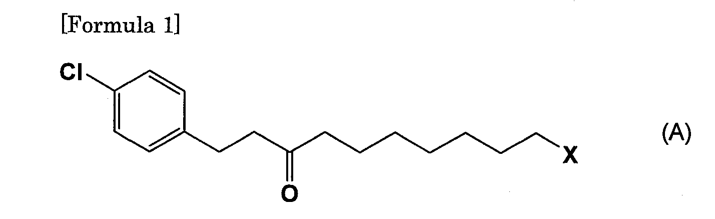 wherein X represents a halogen atom, and a compound represented by the following general formula (B):
CHCl₂COOR¹
wherein R¹ represents hydrogen atom or a protective group of carboxyl group, to prepare a compound represented by the following general formula (C): 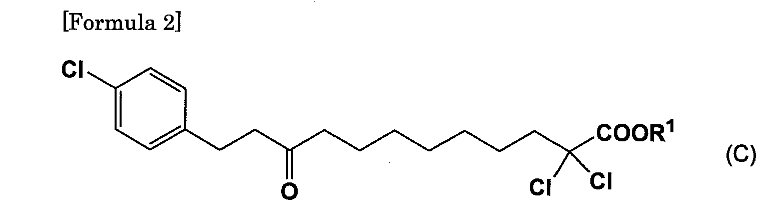 wherein R¹ has the same meaning as that defined above.

2. The method according to claim 1, wherein the step is a step of reacting a compound of the general formula (A) wherein X is iodine atom and a compound of the general formula (B) wherein R¹ is an alkyl group to prepare a compound of the general formula (C) wherein R¹ is an alkyl group.

3. The method according to claim 2, which further comprises the step of reacting a compound of the general formula (A) wherein X is chlorine atom with an iodinating agent to prepare a compound of the general formula (A) wherein X is iodine atom.

4. The method according to claim 2 or 3, which further comprises the step of performing deprotection of the compound of the general formula (C) wherein R¹ is an alkyl group to prepare a compound of the general formula (C) wherein R¹ is hydrogen atom.

5. The method according to claim 4, which further comprises the step of reducing the compound of the general formula (C) wherein R¹ is hydrogen atom to prepare 2,2-dichloro-12-(4-chlorophenyl)-10-hydroxydodecanoic acid.

6. The method according to any one of claims 1 to 5, wherein the compound represented by the general formula (A) is a compound prepared by subjecting a compound represented by the following general formula (D): 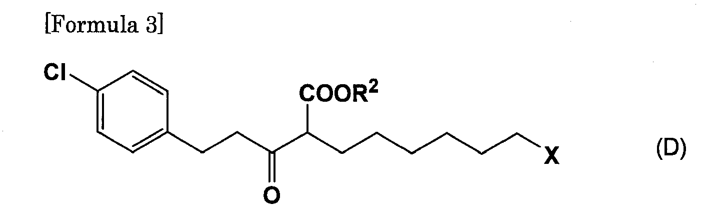 wherein X has the same meaning as that defined above, and R² represents a protective group of carboxyl group, to a decarboxylation reaction.

7. The method according to claim 6, wherein the compound represented by the general formula (D) is a compound obtained by reacting a compound represented by the following general formula (E): 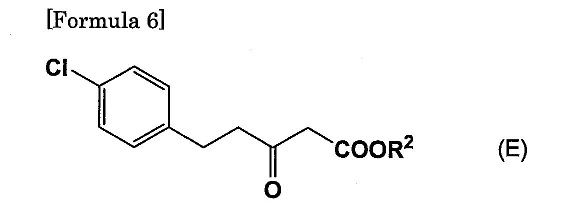 wherein R² has the same meaning as that defined above, with a 1,6-dihalogenohexane.

8. The method according to claim 7, which uses 1-bromo-6-chlorohexane.

9. A method for preparing 2,2-dichloro-12-(4-chlorophenyl)-10-hydroxydodecanoic acid, which comprises the following steps:
(1) the step of reacting a compound represented by the general formula (E) mentioned in claim 7 with 1-bromo-6-chlorohexane in the presence of a base to prepare a compound of the general formula (D) mentioned in claim 6 wherein X is chlorine atom;
(2) the step of decarboxylating the compound obtained in the above step (1) to prepare a compound of the general formula (A) mentioned in claim 1 wherein X is chlorine atom;
(3) the step of reacting the compound obtained in the above step (2) and an iodinating agent to prepare a compound of the general formula (A) mentioned in claim 1 wherein X is iodine atom;
(4) the step of reacting the compound obtained in the above step (3) and a compound of the general formula (B) mentioned in claim 1 wherein R¹ is a protective group of carboxyl group to prepare a compound of the general formula (C) mentioned in claim 1 wherein R¹ is a protective group of carboxyl group;
(5) the step of performing deprotection of the compound obtained in the above step (4) to prepare a compound of the general formula (C) mentioned in claim 1 wherein R¹ is hydrogen atom; and
(6) the step of reducing the compound obtained in the above step (5) to prepare 2,2-dichloro-12-(4-chlorophenyl)-10-hydroxydodecanoic acid.

10. A compound represented by the general formula (A) mentioned in claim 1.

11. The compound according to claim 10, wherein X is chlorine atom or iodine atom.

12. A compound represented by the general formula (D) mentioned in claim 6.

13. The compound according to claim 12, wherein X is chlorine atom, and R² is ethyl group.
